Europäisches Patentamt

European Patent Office

Office européen des brevets

(19)

(11) Numéro de publication : **0 611 824 A1**

## (12) DEMANDE DE BREVET EUROPEEN

(21) Numéro de dépôt : **94400247.6**

(22) Date de dépôt : **04.02.94**

(51) Int. Cl.⁵ : **C12P 19/06,** C09K 7/02, // (C12P19/06, C12R1:64)

(30) Priorité : **16.02.93 FR 9301819**

(43) Date de publication de la demande :
**24.08.94 Bulletin 94/34**

(84) Etats contractants désignés :
**DE DK FR GB IT NL**

(71) Demandeur : **INSTITUT FRANCAIS DU PETROLE**
**4, avenue de Bois Préau**
**F-92502 Rueil-Malmaison (FR)**

(72) Inventeur : **Monot, Frédéric**
**39, rue Parmentier**
**F-92000 Nanterre (FR)**
Inventeur : **Noik, Christine**
**31, Allée de la Capitainerie**
**F-78230 Le Pecq (FR)**
Inventeur : **Ballerini, Daniel**
**103, rue du Pontel**
**F-78100 St Germain en Laye (FR)**

(54) **Composition comprenant un moût de xanthane et des protéines et application de la composition dans un fluide de forage de puits.**

(57)    On décrit une composition d'un moût de xanthane préparée à partir d'un milieu de culture renfermant en particulier au moins une source de soufre de concentration comptée en élément soufre au plus égale à 60 mg par litre de milieu de culture et d'au moins une souche du genre *Xanthomonas*, et d'une source de protéines dans un rapport pondéral protéines sur xanthane compris entre 0,05 et 2. Le moût récupéré contenant de préférence les cellules, à pH alcalin et en présence de cations bivalents, avec les protéines, constitue une composition qui manifeste des propriétés de thixotropie.

   Application de la composition dans un fluide de forage pour le forage des puits.

FIG.2

EP 0 611 824 A1

L'invention concerne une composition contenant un moût de xanthane particulier, en mélange avec une solution enrichie en protéines et l'application de la composition à un procédé de forage mettant en oeuvre un fluide de forage la contenant.

Les fluides de forage jouent un rôle important dans les opérations de forage. Lors d'un forage, la boue de forage est pompée à partir d'un bassin de stockage, injectée dans le train de tiges et remonte par l'espace annulaire situé entre les tiges de forage et les parois du puits.

Les principales fonctions d'un fluide de forage sont de nettoyer le fonds du puits et de remonter les déblais jusqu'à la surface, de maintenir les parois du puits par sa pression hydrostatique, de maintenir les déblais en suspension pendant les arrêts du forage, de stabiliser la formation forée par dépôt d'une fine couche faiblement perméable (le cake), de refroidir l'outil et lubrifier le train de tiges.

Il existe différents types de fluides de forage : les fluides à base d'eau et les fluides à base d'huile qui ont des caractéristiques très variées. Le souci croissant de l'impact sur l'environnement des fluides à base d'huile a conduit au développement des boues à base d'eau.

Les propriété physico-chimiques des fluides de forage sont contrôlées par l'incorporation de différents produits : viscosifiants, réducteurs de filtration, alourdissants, fluidifiants. Les composés entrant majoritairement dans la composition des fluides de forage à base d'eau sont des argiles et des polymères. Les argiles ont une grande affinité pour l'eau et un pouvoir de "gonflement" qui donnent aux suspensions d'argile des propriétés viscosifiantes et augmentent la portance des boues (tenue des déblais en suspension). Les polymères hydrosolubles doivent permettre de maîtriser les principales propriétés du fluide de forage : rhéologie, filtration et stabilisation de la formation. Les critères d'utilisation des polymères concernent essentiellement leur stabilité thermique, mécanique et chimique, qui dépendent des caractéristiques de la formation (pH, salinité, température notamment).

Les polymères couramment utilisés dans les fluides de forage sont les polysaccharides et les polyacrylamides partiellement hydrolysés. Cependant, ces polymères sont très sensibles aux conditions de salinité élevée, en particulier dans le cas de la présence de cations bivalents tels que $Ca^{2+}$ ou $Mg^{2+}$. Il est donc nécessaire de mettre en oeuvre d'autres types de polymères résistants à des conditions de forte salinité. Il a été observé que le xanthane était particulièrement adapté à de telles utilisations du fait de son insensibilité à la présence de sels. La viscosité d'une solution de xanthane diminue peu lorsque la salinité augmente et dans des conditions de salinité élevée, il est thermiquement stable.

L'art antérieur peut être illustré par les documents suivants :
- Enzyme and microbial technology (vol 14, No. 12, Dec. 1992, pages 911 à 996) décrit l'influence de la teneur en soufre du milieu de culture sur la production de xanthane.
- FR 2 331 614 mentionne que l'élément nutritif de limitation dans le milieu de culture conduisant à la production de xanthane est le soufre.
- US 4 400 467 décrit un procédé pour maximiser la production de xanthane à hautre viscosité, dans des conditions limitées en soufre.
- EP-A-259 939 décrit une composition de polysaccharide et notamment de xanthane, et de cations mono- et divalents, dont la stabilité thermique mesurée par sa viscosité est améliorée par l'incorporation de formate.

Une caractéristique supplémentaire recherchée pour les fluides de forage est qu'ils développent de la thixotropie. Cette propriété rend compte de l'aptitude à la formation d'un gel. Ainsi, il est nécessaire que, lors des arrêts de circulation des fluides de forage, comme il peut s'en produire au cours d'un forage, les déblais forés ne se redéposent pas au fonds du puits car ils provoqueraient un blocage de l'outil de forage au redémarrage de celui-ci. Or, cette propriété est absente des fluides à base d'eau contenant le xanthane habituellement utilisé. Il est alors nécessaire d'ajouter d'autres produits comme des argiles de type bentonite pour améliorer la thixotropie des boues. L'utilisation des argiles est tout de même limitée car elle augmente fortement le coût du forage compte tenu des volumes de fluide mis en oeuvre, en général de 20 à 60 kg/m³, et d'autre part, elle nécessite le stockage de grandes quantités de produit sur le lieu du forage. De plus, les bentonites perdent de leur activité en milieu salé.

Un des objets de l'invention est de remédier aux inconvénients de l'art antérieur. L'invention a donc pour objet la production d'un xanthane qui, dans des conditions particulières d'utilisation, et en particulier dans les conditions de forage où il est préconisé, c'est-à-dire à salinité et à pH élevés, présente un caractère thixotrope, notamment quand il est associé à une source de proteines.

De manière détaillée, le moût de xanthane ayant des propriétés améliorées est produit à partir d'un milieu de culture contenant, au moins une source de carbone organique, au moins une source d'azote, au moins une source de phosphore et au moins une source de potassium, et d'au moins une souche bactérienne du genre Xanthomonas en présence d'air et dans des conditions de température et de pH appropriées. Le milieu de culture renferme en outre au moins une source de soufre de concentration comptée en élément soufre au plus égale à 60 mg par litre de milieu de culture. On récupère un moût de fermentation contenant le xanthane.

Par moût de fermentation contenant le xanthane, ou entend le milieu de culture contenant le produit après fermentation, dilué ou concentré, ou le xanthane issu de ce milieu.

Le xanthane est produit par fermentation de Xanthomonas sur un milieu de culture caractérisé en tout instant par sa faible teneur en soufre. Le moût de fermentation contenant les cellules, mis en présence de cations appropriés (eau de mer par exemple) et à un pH alcalin, présente un caractère thixotrope, alors que les xanthanes habituellement utilisés, c'est-à-dire issus de cultures sans limitation en soufre, mis dans les mêmes conditions, ne développent pas de thixotropie. Il est avantageux d'utiliser un moût non raffiné, c'est-à-dire un milieu contenant une partie au moins des cellules de *Xanthomonas*, et de préférence la totalité des cellules, pour développer de la thixotropie.

Selon l'invention, la composition comprend aussi au moins une source de protéines, pouvant être issues de tissus animaux ou végétaux ou bien de protistes, qui peut être ajoutée à la solution ci-dessus contenant le xanthane pour renforcer notamment la dureté du gel. Cette source peut consister en un mélange de sources de protéines ou bien en un seul des composés. Ceux-ci peuvent être par exemple de l'extrait de levure, de l'extrait de malt, de la liqueur de macération de maïs (corn steep), des extraits de distillats de soja, de l'extrait de viande, des peptones de caséine, de pancréas, de viande, de soja ou autres, des extraits de soja, de gluten, des farines riches en protéines telles que farines de poisson, de viande, de sojas. Ces sources de proteines peuvent être sous forme de poudre ou de solution liquide. Elles sont rajoutées habituellement au moût de xanthane dans un rapport généralement compris entre 0,05 et 2 en poids, de façon avantageuse dans un rapport de 0,1 à 1,5 en poids et de préférence de 0,25 à 0,80 en poids par rapport au xanthane, la concentration en xanthane étant déterminée selon des méthodes connues de l'homme de l'art.

Le mélange riche en protéines peut être rajouté dans les mêmes proportions à la solution ci-dessus contenant de préférence le xanthane non raffiné comportant les cellules de la souche, c'est-à-dire après que le moût de fermentation ait été précipité par au moins un alcool (par exemple du méthanol, éthanol ou isopropanol) ou par au moins une cétone (par exemple de l'acétone) de préférence en présence de sel (par exemple du chlorure de potassium), puis séché jusqu'à l'obtention d'une poudre. Le mélange riche en protéines peut aussi être rajouté dans les mêmes proportions à la solution ci-dessus contenant le moût de fermentation une fois concentré par ultrafiltration ou évaporation. On obtient dans ces conditions un gel de qualité améliorée.

Le milieu de culture ou de fermentation en règle générale comprend d'une part un substrat carboné d'origine organique, de préférence un substrat contenant des hydrates de carbone tels que glucose, fructose ou saccharose, provenant ou non de l'hydrolyse acide ou enzymatique de substrats sucrés dans des conditions connues de l'homme de métier. Il comprend également un substrat azoté, de préférence fourni sous forme minérale ammoniacale telle que le chlorure d'ammonium. Il peut comprendreaussi de l'azote sous forme organique, par exemple par apport d'acides aminés à condition que l'incorporation de la source d'azote organique n'entraîne pas un apport trop élevé de soufre. Cet azote organique peut être aussi dilué par de l'azote d'origine minérale. Il contient également du phosphore et du potassium fournis sous forme minérale. Le soufre peut être fourni sous forme organique ou minérale, de préférence sous forme minérale, par exemple sous forme de sulfate. La concentration en soufre du milieu de fermentation est en général inférieure a 60 milligrammes par litre, par exemple comprise entre 0,1 et 60 mg/l avantageusement entre 0,3 et 30 mg/l et de préférence entre 0,5 et 15 mg/l, comptée en élément soufre. Habituellement, la concentration en sucre est comprise entre 1 et 100 g/l de milieu de culture, de préférence entre 2 et 50 g/l et de façon préférée entre 5 et 30 g/l. Le rapport pondéral C/N peut se situer par exemple aux alentours de 5, mais peut varier dans une large gamme sans que cela ne modifie les propriétés du xanthane obtenu. Certains éléments comme le magnésium et des oligoéléments peuvent entrer dans la composition du milieu. Il peut être avantageux d'introduire de l'extrait de levure, à des concentrations faibles par exemple inférieures à 1 g/l et de préférence comprises entre 0,1 et 0,5 g/l.

Le milieu est soumis à la fermentation par une souche appartenant au genre *Xanthomonas,* telle que par exemple *Xanthomonas campestris* ou une de ses sous-espèces, *Xanthomonas albilineans*, *Xanthomonas fragariae* ou *Xanthomonas graminis.*

La fermentation est en général conduite à un pH compris entre 5 et 8 et à une température comprise entre 20 et 40°C. Le milieu est aéré et de préférence agité. Les conditions de fermentation sont : 25 à 33°C, pH entre 6,5 et 7,5, aération entre 0,2 et 1 volume d'air par volume liquide de milieu et par minute (vvm).

Ces conditions peuvent être modifiées en fonction du déroulement de la fermentation et en fonction de la taille du fermenteur. Dans le cas d'une fermentation continue, après ensemencement du milieu par une préculture de Xanthomonas réalisée en général sur un milieu identique au milieu du fermenteur, le réacteur est alimenté par un milieu d'alimentation à un débit identique au débit de soutirage afin de garder un poids (ou un volume, selon le mode de régulation de niveau choisi) constant. Le taux de dilution qui est égal au rapport du débit d'alimentation (ou de soutirage) sur le volume réactionnel au sein du fermenteur est compris en général entre 0,01 et 0,3 h⁻¹, et de préférence compris entre 0,02 et 0,1 h⁻¹. Le moût de fermentation récolté contenant les cellules peut être thermisé ou non. il peut aussi être précipité et séché ou il peut être concentré comme il est précisé ci-après.

Pour observer le phénomène de thixotropie, le moût obtenu, brut par exemple, est ensuite amené à un pH alcalin, de préférence compris entre 8 et 11 avec un agent alcalin comme la soude ou la potasse, mis en présence de sels, en particulier d'ions bivalents, tels que calcium, magnésium ou manganèse, à une concentration totale en cations bivalents en général au moins égale à 0,5 g/l, par exemple 0,5 à 20 g/l, avantageusement comprise entre 1 et 15 g/l et de préférence comprise entre 2 et 10 g/l. Ces cations bivalents peuvent être sous la forme de sels d'acides minéraux et/ou carboxyliques. Il est par exemple possible d'utiliser l'eau de mer comme agent de dilution.

La mesure de la contrainte de cisaillement à différentes vitesses de cisaillement s'effectue avec un viscosimètre Rotovisco RV20 de Haake. Ces valeurs sont mesurées d'abord en augmentant la vitesse de cisaillement puis en la diminuant. La non juxtaposition des deux courbes obtenues est caractéristique d'un comportement thixotrope.

Dans certains cas, la dureté du gel a été évaluée par mesure de la valeur de gel. Celle ci est la différence entre les contraintes de cisaillement mesurées à faible contrainte de cisaillement après un repos de 10 minutes.

L'invention concerne enfin l'utilisation d'un fluide de forage comprenant une quantité utile de xanthane préparé selon l'invention et contenu dans la composition décrite ci-avant, avec la solution enrichie en protéines, pour le forage de puits. La quantité de xanthane nécessaire à l'obtention du fluide de forage peut être comprise entre 0,5 et 20 g/l et de préférence entre 1 et 10 g/l de solution finale du fluide. Celle-ci peut comprendre d'autres produits comme des dérivés cellulosiques qui jouent un rôle de réducteur de filtration, ces produits ne devant pas modifier les conditions d'application de la composition finale.

La mise en oeuvre du fluide de forage contenant la composition selon l'invention est réalisée de manière connue par l'homme de l'art.

Le caractère thixotrope de la composition contenant le xanthane préparé selon l'invention permet d'utiliser celui-ci en tant qu'agent thixotrope dans les fluides de forage et de limiter au moins en partie l'ajout d'argiles. Dans les conditions de salinité et de pH préconisées, le xanthane reste un bon agent viscosifiant. Son utilisation est particulièrement intéressante quand les eaux de base du fluide de forage sont une eau de mer, une saumure ou tout autre type d'eau dure, par exemple calcaire ou magnésienne alors que dans ces conditions les argiles sont beaucoup moins actives que dans des eaux douces.

L'invention sera mieux comprise au vu des exemples illustratifs suivants, dans lesquels :

- Les figures 1 et 6 représentent les contraintes de cisaillement exprimées en pascals (Pa) en fonction de la vitesse de cisaillement exprimée en s⁻¹ pour des moûts de fermentation contenant du xanthane préparé sans limitation en soufre.
- Les figures 2 à 5 montrent les mêmes représentations pour des moûts de xanthane selon l'invention, dans des conditions de salinité et de pH différentes, avec ou sans protéines.

**Exemple 1 : Préparation de xanthane selon l'art antérieur**

La souche de *Xanthomonas campestris* DSM 1706 utilisée est conservée à - 80°C sur milieu complexe gélosé. Pour la remise en culture de cette souche, un tube est remis à la température ambiante pendant quelques heures et les colonies présentes en surface remises en suspension dans environ 1 ml du milieu stérile dont la composition est décrite ci-dessous. Cette suspension sert à ensemencer des fioles contenant 100 ml de milieu de culture stérile constitué de :

| | | |
|---|---|---|
| Glucose | : 10 | g.l⁻¹ |
| Extrait de levure | : 3 | g.l⁻¹ |
| Extrait de malt | : 3 | g.l⁻¹ |
| Peptone | : 5 | g.l⁻¹ |
| K2HPO4 | : 5,0 | g.l⁻¹ |
| MgSO₄, 7H₂O | : 0,5 | g.l⁻¹ |

Ce milieu est dit complexe car il contient majoritairement des sources d'azote et d'autres éléments d'origine organique. La teneur en soufre de ce milieu peut être estimée à environ 200 mg.l$^{-1}$ et il est majoritairement fourni par les composés amenant l'azote sous forme organique. C'est ce type de milieu qui est utilisé le plus souvent pour les fermentations industrielles. Le pH de ce milieu est ajusté à 7 avec NaOH en pastilles. Le milieu est ensuite mis à incuber à 30°C pendant 24 heures et agité. Deux fioles de 100 ml de ce milieu servent à ensemencer 3,5 litres d'un milieu de composition identique. Cette culture est effectuée selon un mode discontinu, c'est-à-dire que le milieu n'est pas renouvelé au sein du fermenteur, dans un fermenteur LSL-Biolafitte de 6 litres de volume total, que l'on agite. La vitesse d'agitation est maintenue à 600 tr/min tout au long de la fermentation. Le pH est mesuré par une sonde stérilisable et il est régulé à 6,9 par l'intermédiaire d'un régulateur qui commande l'injection de KOH 5 N dans le milieu de culture. L'aération s'effectue par injection d'air en-dessous de la turbine d'agitation, inférieure à un débit de 0,5 vvm. Le fermenteur est également équipé d'une sonde Biolafitte mesurant la pression en oxygène dissous. La sortie d'air du fermenteur est connectée à un analyseur de $CO_2$.La température est maintenue à 25°C tout au long de la culture.

Au cours de cette fermentation sont suivis et enregistrés en continu la pression d'oxygène dissous, le taux de $CO_2$ dans le gaz de sortie, et le volume de base injecté pour maintenir le pH à la valeur désirée. Des prélèvements de 10 ml environ sont effectués régulièrement. Ces échantillons de moûts de fermentation servent à estimer la concentration bactérienne, la concentration en xanthane formé, la concentration en glucose résiduel et la viscosité apparente. La concentration bactérienne est estimée par mesure de la densité optique à 600 nm. La concentration en xanthane est déterminée par mesure du poids sec après précipitation du polymère par un solvant organique. Cette méthode consiste à ajouter 10 ml d'éthanol absolu et 10 ml d'acétone à 10 ml de moût de fermentation. On ajoute ensuite 3 gouttes de KCl saturé et l'on agite le mélange que l'on laisse en contact pendant au moins 15 minutes. L'ensemble est ensuite filtré sur une membrane GF/C Whatman préalablement tarée, puis lavé deux fois avec de l'acétone avant d'être séché sur une balance Mettler équipée d'un dispositif de séchage par infra-rouge jusqu'à obtention d'un poids constant. La concentration en xanthane est ensuite exprimée par litre de moût de fermentation.

Les autres analyses sont effectuées sur le surnageant obtenu après centrifugation de l'échantillon. La concentration en glucose libre dans le milieu est déterminée par dosage enzymatique réalisé au Glucostat (analyseur automatique de glucose Beckman). La viscosité apparente du moût de fermentation est estimée par écoulement dans des tubes capillaires pré-calibrés. Les mesures du temps d'écoulement sont effectuées dans des tubes choisis pour que ce temps soit supérieur à 3 minutes. Il suffit ensuite d'appliquer la formule $\eta = K'\rho\, t$ où $\eta$ est la viscosité apparente en mPa.s, K' la constante d'étalonnage du tube donnée par le fabricant, $\rho$ la masse volumique du liquide en g/cm$^3$ (prise égale à 1) et t le temps d'écoulement entre les deux repères en secondes.

Le tableau 1 donne l'évolution des paramètres de fermentation au cours de la culture sur milieu complexe, riche en soufre. La fermentation a été arrêtée au moment où la teneur en glucose dans le milieu est devenue nulle.

**Tableau 1**

| Temps | $O_2$ dissous | $CO_2$ gazeux | Densité optique | Glucose | Viscosité apparente | Xanthane |
|---|---|---|---|---|---|---|
| (h) | (%) | (%) | | (g/l) | mPa.s | (g/l) |
| 0 | 95 | 0 | 0,24 | 30 | 1 | |
| 19 | ≈0 | 11,3 | 7,06 | 24 | 11 | 5,9 |
| 26 | 0 | > 12 | 9,85 | 20 | 54 | |
| 43 | 20 | 9,2 | 12,10 | 9 | 1880 | 12 |
| 50 | 30 | 8,2 | 11,80 | 6 | 1900 | |
| 67 | | 5,0 | 13,00 | 0 | 4000 | 15 |

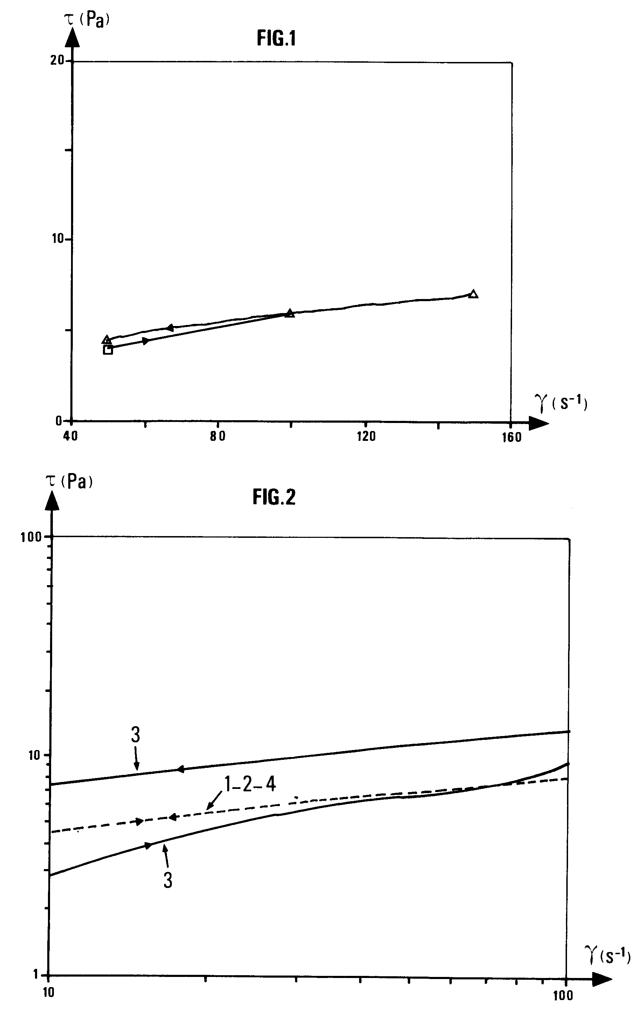

Le moût brut final est ensuite caractérisé par son comportement rhéologique. Dans ce cas, on a cherché à mettre en évidence l'existence de comportements thixotropes des moûts obtenus ou additionnés d'autres substances. Ces essais de caractérisation ont été effectués sur un viscosimètre Rotovisco RV20 de Haake. Les courbes d'écoulement, c'est-à-dire la contrainte de cisaillement en fonction de la vitesse de cisaillement, ont été réalisées dans le sens d'une augmentation de la vitesse puis dans le sens d'une diminution. L'existence d'un comportement thixotrope se manifeste par la non-juxtaposition des deux courbes obtenues. Dans le cas de solutions gélifiées, pour obtenir des valeurs représentatives et exploitables, il a fallu attendre suffisamment longtemps (pour certaines solutions, plus d'une heure) pour que la valeur lue soit bien stable. Dans ce cas, on a limité nos mesures à trois valeurs de vitesses de cisaillement, 50, 100 et 150 $s^{-1}$. Le moût a été ramené à une concentration de 7 $g.l^{-1}$ par dilution dans une solution d'eau de mer reconstituée à laquelle on a rajouté un mélange riche en protéines, la solution finale étant ensuite ajustée à pH 8 par ajout de KOH 4 N. Le mélange dit protéique est préparé en mélangeant du corn steep sous forme de poudre et de l'extrait de levure à raison de 75 % de corn steep et 25 % d'extrait de levure (poids/poids) dans de l'eau distillée. Cette solution est ensuite centrifugée et seul le surnageant est conservé. Celui-ci est rajouté au moût dans un rapport 0,5 en poids par rapport au xanthane. Par exemple, à une solution de 7 $g.l^{-1}$ de xanthane, on incorpore le mélange 75/25 de corn steep/extrait de levure de façon à être à 3,5 g/l de concentration finale de ce mélange, le xanthane étant dilué au minimum. La dilution dans l'eau de mer consiste en réalité à incorporer des sels dans une proportion telle que le xanthane se retrouve finalement en solution dans une eau de mer reconstituée. La composition d'eau de mer utilisée est la suivante : 27,80 $g.l^{-1}$ NaCl; 9,54 $g.l^{-1}$ $MgCl_2$, $6(H_2O)$; 7,73 $g.l^{-1}$ $Na_2SO_4$, $10(H_2O)$; 1,62 $g.l^{-1}$ $CaCl_2$, 2 $(H_2O)$. On observe que, dans ces conditions, les valeurs des contraintes de cisaillement obtenues aux différentes vitesses de cisaillement imposées sont quasiment identiques dans le sens de la montée ou dans celui de la descente, donnant deux courbes sensiblement juxtaposables (figure 1). Ce résultat montre que le xanthane produit, mis en solution dans les conditions mentionnées, a fortiori les plus favorables au développement d'une thixotropie, ne présente pas de comportement thixotrope marqué.

## Exemple 2 : Préparation selon l'invention

Dans cet exemple, la souche est identique à celle de l'exemple 1. La remise en culture de celle-ci s'effectue selon le même protocole que celui de l'exemple 1. Pareillement, deux fioles de 100 ml de milieu stérile sont ensemencées et mises a incuber à 30°C sur un plateau rotatif. Le milieu utilisé a la composition suivante :

| Glucose | : 10 | $g.l^{-1}$ |
|---|---|---|
| Extrait de levure | : 0,1 | $g.l^{-1}$ |
| NH4Cl | : 0,4 | $g.l^{-1}$ |
| $H_3PO_4$ 85 % | : 0,5 | $ml.l^{-1}$ |
| $K_2HPO_4$ | : 2,0 | $g.l^{-1}$ |
| $Na_2SO_4$ | : 0,05 | $g.l^{-1}$ |
| $MgCl_2$, 6 $H_2O$ | : 0,1 | $g.l^{-1}$ |
| $CaCl_2$ | : 23,0 | $mg.l^{-1}$ |
| $H_3BO_3$ | : 6,0 | $mg.l^{-1}$ |
| $ZnCl_2$ | : 7,0 | $mg.l^{-1}$ |
| $FeCl_3$, 6 $H_2O$ | : 2,4 | $mg.l^{-1}$ |

Contrairement à celui utilisé dans l'exemple 1, ce milieu est un milieu synthétique parfaitement défini chimiquement (hormis les éléments apportés par 0,1 $g.l^{-1}$ d'extrait de levure qui sont négligeables par rapport à l'apport de substrats complexes du milieu de l'exemple 1). Ces fioles servent à ensemencer un fermenteur identique à celui de l'exemple précédent et contenant 3 litres du même milieu que celui utilisé dans les fioles. La température du réacteur est de 25°C, la vitesse d'agitation de 600 tr/min, l'aération de 0,05 vvm. Le pH est régulé à 6,9 par injection de KOH 5 N. Après un batch préliminaire de 40 heures permettant d'augmenter la concentration cellulaire,est démarrée une fermentation continue. Le milieu d'alimentation est le même que celui décrit ci-dessus. Ce milieu a été choisi principalement pour sa faible concentration en soufre (11 $mg.l^{-1}$) qui permet d'effectuer une fermentation continue avec le soufre comme élément limitant. Le niveau de liquide dans le fermenteur est maintenu constant grace à un régulateur de pression hydrostatique qui commande une pompe de soutirage. Le débit d'injection est fixé de manière à avoir un taux de dilution égal à 0,02 $h^{-1}$, le taux de dilution étant défini comme le rapport du débit d'alimentation (égal au débit de soutirage) sur le volume de liquide dans le fermenteur. Le moût de fermentation ayant été utilisé pour les essais de détermination de la rhéologie, a été récolté entre la 137ème et la 185ème heure de culture. A ce moment, les paramètres de fermentation sont approximativement les suivants :

| O$_2$ dissous | CO$_2$ gazeux | Densité optique | Glucose | Viscosité apparente | Xanthane |
|---|---|---|---|---|---|
| (%) | (%) | | (g/l) | mPa.s | (g/l) |
| 45 | 0,80 | 2,93 | 1,0 | 300 | 5,35 |

Ce moût de fermentation contenant les cellules de *Xanthomonas* a été amené à pH 7 ou à pH 8 dans de l'eau de mer reconstituée dans des conditions identiques à celles décrites dans l'exemple 1 ou dans de l'eau contenant 5 g.l$^{-1}$ de NaCl. Les variations de la contrainte de cisaillement ($\tau$) en fonction de la vitesse de cisaillement ($\gamma$) lors de l'augmentation et de la diminution de celle-ci sont indiquées dans la figure 2. On constate que, dans la solution à 5 g.l$^{-1}$ NaCl, quel que soit le pH, 7 ou 8, les courbes d'écoulement (1 et 2) sont identiques et indépendantes du sens de variation de la vitesse de cisaillement. Dans l'eau de mer, alors qu'à pH 7 les deux courbes (4) se superposent, à pH 8 la viscosité du moût ne varie pas de la même façon lors de l'augmentation de la vitesse de cisaillement ou lors de sa diminution (courbes 3), démontrant un comportement thixotrope de cette solution. Ce comportement thixotrope ne se manifeste donc que dans des conditions bien particulières de pH et de salinité. La présence d'ions bivalents tels que le calcium et le magnésium présents dans l'eau de mer est nécessaire pour que ce moût de fermentation présente un comportement thixotrope. Il est de plus nécessaire de se placer à un pH supérieur à 7 pour que le comportement thixotrope apparaisse.

**Exemple 3 : Préparation d'un moût sur un milieu à 50 mg/l de soufre**

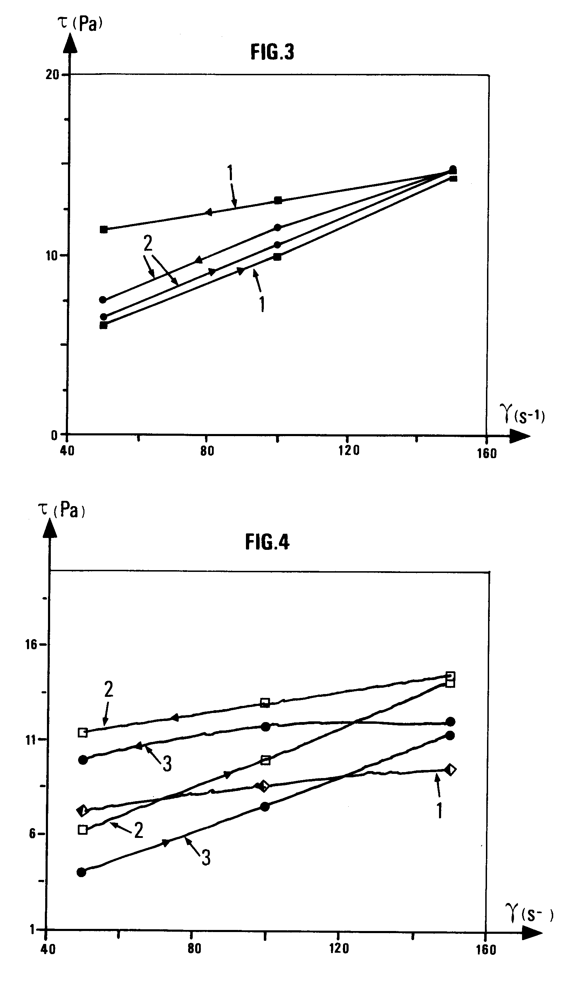

Un moût de xanthane est préparé dans les mêmes conditions que celles de l'exemple 2 à l'exception de la concentration en soufre du milieu de fermentation qui correspond à 50 mg/l de milieu de culture compté en élément soufre. Le moût de fermentation récolté obtenu a ensuite été traité de façon identique à celle décrite dans l'exemple 2, c'est-à-dire qu'il a été amené à pH 8 et dans de l'eau de mer. Les valeurs des contraintes de cisaillement ($\tau$) ont été mesurées à des vitesses de cisaillement ($\gamma$) de 50, 100, 150 s$^{-1}$ sur les deux solutions une fois qu'une valeur stable ait été obtenue. Les courbes de la figure 3 montrent que le moût obtenu selon l'exemple 2 (courbe 1), c'est-à-dire issu d'un milieu de culture contenant 10 mg/l de soufre a un caractère thixotrope très marqué alors que le moût de xanthane obtenu selon l'exemple 3 (courbe 2) qui est issu d'un moût de fermentation contenant 50 mg/l de soufre a un caractère thixotrope moins marqué.

**Exemple 4 : Influence de l'ajout de protéines**

Afin de mieux cerner l'influence de l'ajout de protéines sur la rhéologie des moûts de polysaccharides, on a pris le moût issu de la fermentation décrite dans l'exemple 2 auquel on a rajouté un mélange riche en protéines de façon identique à celle décrite dans l'exemple 1. Cet ajout de protéines a été effectué dans de l'eau salée à 5 g.l$^{-1}$ en NaCl et dans de l'eau de mer reconstituée, ces solutions étant ajustées à pH 8. Les valeurs des contraintes de cisaillement à 50, 100 et 150 s$^{-1}$ lues après stabilisation dans le sens de la montée et de la descente des vitesses de cisaillement sont indiquées dans la figure 4.

Quand le solvant est de l'eau contenant 5 g.l$^{-1}$ NaCl (courbe 1), l'addition de protéines ne modifie pas le comportement de la solution de xanthane qui reste de type pseudoplastique et ne développe pas de thixotropie. Dans l'eau de mer et à pH 8, les valeurs de contraintes (courbe 2) ne sont pas les mêmes selon que l'on augmente ou que l'on diminue la vitesse de cisaillement. Cet effet est sensiblement exalté quand on ajoute des protéines (courbe 3). De plus, on a visuellement observé que les gels de xanthane formés en présence de protéines (et d'eau de mer et à pH 8) avaient une consistance beaucoup plus compacte que les gels formés sans apport supplémentaire de protéines.

Afin de mieux estimer le gain éventuel amené par l'addition de protéines, on a déterminé les "valeurs de gel". Celles-ci sont évaluées par les valeurs de contraintes obtenues de la façon suivante. La solution est soumise pendant 30 secondes à une vitesse de cisaillement de 2700 s$^{-1}$, puis laissée au repos pendant 10 secondes et la contrainte est ensuite mesurée à 4,49 s$^{-1}$ : c'est la contrainte $\tau_1$. La solution est ensuite de nouveau amenée à 2700 s$^{-1}$ pendant 30 secondes, laissée au repos pendant 10 minutes cette fois et la deuxième valeur de la contrainte à 4,49 s$^{-1}$ ($\tau_2$) est lue. La "valeur de gel" est donnée par l'écart entre les deux mesures, et plus cet écart est grand, plus la tendance à former un gel est forte.

Pour ces essais, on a utilisé le moût d'une fermentation menée dans des conditions similaires à celles décrites dans l'exemple 2. Ce moût a été testé selon le protocole indiqué ci-dessus en le mettant dans différentes conditions: addition supplémentaire de protéines, pH alcalin, présence d'eau de mer. La solution protéique utilisée pour le rajout consiste, comme pour les autres tests, en un mélange 75/25 de corn steep et d'extrait de levure, cette solution étant centrifugée préalablement à son incorporation et étant ajoutée dans un rapport de 0,5 ou 1 en poids par rapport au xanthane. Le pH a été ajusté à pH 10 à l'aide de KOH 4 N. Les échantillons ont été laissés pendant 5 jours à température ambiante avant les mesures des valeurs de gel effectuées à 30°C. Celles-ci sont indiquées dans le tableau 2. On a constaté que la solution dont la dureté est la plus forte est la solution contenant des protéines, dans l'eau de mer et à pH 10. Il y a en effet pour cet échantillon une grande différence entre la première et la seconde lecture. Ces résultats montrent donc que la présence de protéines conjuguée à la présence d'eau de mer et à un pH alcalin induit un processus d'agrégation du xanthane produit selon les conditions sus-mentionnées.

### Exemple 5 : Influence de la présence de cellules

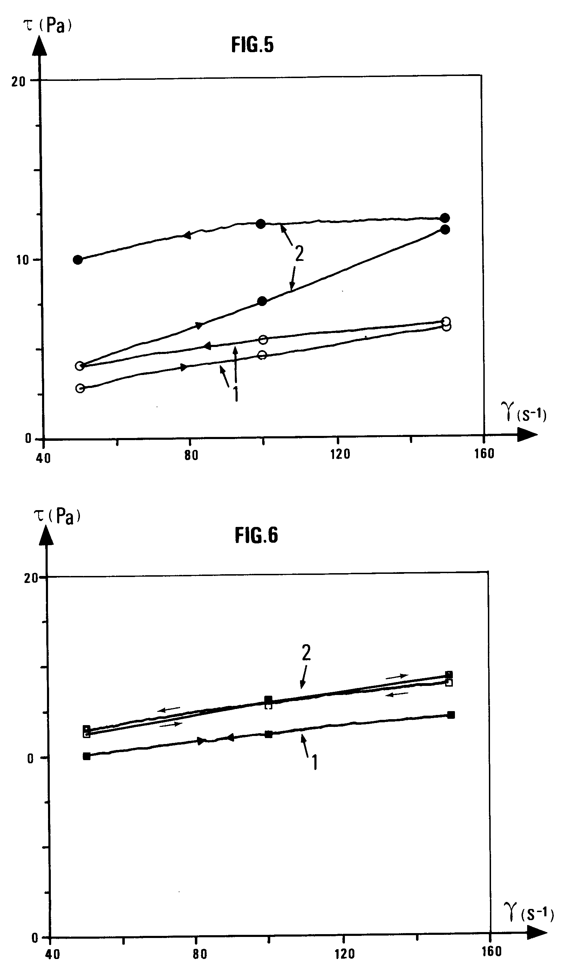

L'échantillon de xanthane utilisé dans les essais précédents est un moût brut contenant donc les cellules de *X. campestris* issues de la fermentation. Afin de voir si les cellules elles-mêmes peuvent jouer un rôle dans le processus d'agrégation du xanthane, on a tenté de clarifier au mieux le moût issu de la fermentation décrite dans l'exemple 2 en le centrifugeant pendant 11 heures à 50.000 g. Le surnageant obtenu est limpide et on peut donc penser que la plus grande partie des cellules a été éliminée. A cette solution clarifiée, on a rajouté des protéines de la même manière et aux mêmes doses que décrit dans les exemples 1 et 3, puis reconstitué la solution dans de l'eau de mer et enfin ajusté le pH à 8. Les viscosités ont été déterminées à 50, 100 et 150 $s^{-1}$ et celles-ci sont présentées sur la figure 5. La solution centrifugée (courbe 1) développe peu de thixotropie. Il apparaît donc que les cellules jouent un rôle important dans l'apparition d'un comportement thixotrope. Il est avantageux d'avoir un moût brut non centrifugé (courbe 2) pour que cette thixotropie soit la plus manifeste possible.

### Tableau 2 :
### "Valeurs de gel" des solutions de moûts de xanthane

| pH | Salinité | [Protéines][a] | 1ère contrainte ($\tau_1$ en Pa) | 2ème contrainte ($\tau_2$ en Pa) | D ($\tau_1$-$\tau_2$) |
|---|---|---|---|---|---|
| 7 | 5 g.l$^{-1}$ NaCl | 0 | 4,8 | 5,0 | 0,2 |
| 7 | 5 g.l$^{-1}$ NaCl | 0,5 | 3,2 | 3,8 | 0,6 |
| 7 | 5 g.l$^{-1}$ NaCl | 1,0 | 4,3 | 4,1 | 0,2 |
| 10 | 5 g.l$^{-1}$ NaCl | 0,5 | 3,1 | 3,6 | 0,5 |
| 7 | eau de mer | 0 | 3,2 | 4,3 | 1,1 |
| 7 | eau de mer | 0,5 | 4,1 | 4,5 | 0,4 |
| 10 | eau de mer | 0 | 10,3 | 9,5 | 0,8 |
| 10 | eau de mer | 0,5 | 0,6 | 3,8 | 3,2 |

[a]Solution protéique de corn steep et extrait de levure - Rapport en poids par rapport au xanthane

### Exemple 6 : Essais avec des xanthanes commerciaux

Des mesures de contraintes de cisaillement à des vitesses de cisaillement de 50, 100 et 150 $s^{-1}$ d'abord

dans le sens de la montée puis dans le sens de la descente ont été effectuées en utilisant un moût de xanthane concentré Pfizer (4800 CX) (courbe 1) et une poudre de xanthane Rhône-Poulenc (RP 23) (courbe 2), tous ces échantillons étant ramenés à une concentration en xanthane identique. Les mesures ont été faites sur les solutions dans l'eau de mer, à pH 8 et en présence des mêmes protéines. Les résultats sont présentés sur la figure 6. On constate que ces échantillons, placés dans des conditions identiques à celles dans lesquelles le xanthane préparé selon l'exemple 2 et en présence de protéines présente un comportement thixotrope, ne manifestent pas de caractère thixotrope marqué. Ces derniers exemples montrent le caractère particulier du xanthane préparé dans des conditions où la teneur en soufre est limitée.

**Revendications**

1. Composition contenant :
   - un moût de fermentation comprenant du xanthane produit à partir d'un milieu de culture contenant au moins une source de carbone organique, au moins une source d'azote, au moins une source de phosphore, au moins une source de potassium, au moins une source de soufre de concentration comptée en élément soufre au plus égale à 60 mg par litre de milieu de culture, et d'au moins une souche bactérienne du genre Xanthomonas en présence d'air ; et
   - au moins une source de protéines, dans un rapport pondéral protéines sur xanthane compris entre 0,05 et 2.

2. Composition selon la revendication 1, dans laquelle la souche bactérienne appartient à l'espèce *Xanthomonas campestris* ou à une de ses sous-espèces, *Xanthomonas albilineans, Xanthomonas fragariae* ou *Xanthomonas graminis.*

3. Composition selon l'une des revendications 1 à 2, dans laquelle le moût de fermentation est préparé en continu, avec un taux de dilution de 0,01 à 0,3 $h^{-1}$ et de préférence compris entre 0,02 et 0,1 $h^{-1}$.

4. Composition selon l'une des revendications 1 à 3, dans laquelle la source de carbone est du glucose à une concentration de 1 a 100 g/l de milieu de culture, la source d'azote est sous forme minérale ammoniacale, la source de soufre est à une concentration de 0,3 à 30 mg/l de milieu de culture, la souche bactériennne est *Xanthomonas campestris* et dans lequel la température est de 20 à 40°C et le pH de 5 à 8.

5. Composition selon l'une des revendications 1 à 4, dans laquelle le moût de fermentation contient au moins une partie des cellules *Xanthomonas* et de préférence la totalité des cellules.

6. Composition selon l'une des revendications 1 à 5, dans laquelle le moût de fermentation est précipité par au moins un alcool ou au moins une cétone pour récupérer après séchage, du xanthane non raffiné contenant les cellules.

7. Composition selon l'une des revendications 1 à 5, dans laquelle le moût de fermentation est concentré par ultrafiltration ou évaporation.

8. Composition selon l'une des revendications 1 à 7, dans laquelle les protéines sont d'origine animale, végétale ou issues de protistes et de préférence un extrait de macération du maïs et/ou un extrait de levure.

9. Procédé de forage d'un puits dans lequel on utilise un fluide de forage contenant la composition en solution qui comporte une quantité utile de xanthane selon l'une des revendications 1 à 8, en présence de cations bivalents à une concentration d'au moins 0,5 g/l de solution et à pH alcalin, pour développer de la thixotropie.

10. Procédé selon la revendication 9, dans lequel la quantité utile de xanthane est comprise entre 0,5 et 20 g/l, de préférence entre 1 et 10 g/l.

11. Procédé selon l'une des revendications 9 à 10, dans lequel les cations bivalents sont apportés par de l'eau de mer.

FIG.1

FIG.2

τ (Pa)

**FIG.3**

20

10

γ(s⁻¹)

0

40    80    120    160

τ (Pa)

**FIG.4**

16

11

6

1

γ(s⁻)

40    80    120    160

FIG.5

FIG.6

EP 0 611 824 A1

Office européen
des brevets

# RAPPORT DE RECHERCHE EUROPEENNE

Numéro de la demande

EP 94 40 0247

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| D,A | ENZYME AND MICROBIAL TECHNOLOGY, vol.14, no.12, Décembre 1992 pages 991 - 996 F. GARCIA-OCHOA ET AL. 'Nutritional study of Xanthomonas campestris in xanthan gum production by factorial design of experiments' * page 991, colonne de gauche, alinéa 1 * * page 991, colonne de droite, dernier alinéa - page 992, colonne de droite, alinéa 4; tableaux 1,2,9 * * page 994, colonne de gauche, dernier alinéa - page 995, colonne de gauche, alinéa 1 * * page 995, colonne de droite, alinéa 3 * --- | 1,2,6-8 | C12P19/06 C09K7/02 //(C12P19/06, C12R1:64) |
| D,A | FR-A-2 331 614 (UNITED KINGDOM OF GREAT BRITAIN AND NORTHERN IRELAND) * page 2, ligne 6 - page 3, ligne 6 * * page 4, ligne 5 - page 5, ligne 6 * * page 5, ligne 16 - ligne 34 * * page 6, ligne 35 - page 7, ligne 3; exemples 5-15 * --- | 1-3,6 | |
| D,A | US-A-4 400 467 (KEITH A. BAUER ET AL.) * colonne 2, ligne 32 - colonne 3, ligne 58 * * colonne 4, ligne 5 - ligne 12; exemple II * --- | 1,2,4 | |
| D,A | EP-A-0 259 939 (SHELL INTERNATIONALE RESEARCH MAATSCHAPPIJ B.V.) * page 2, ligne 1 - ligne 8 * * page 2, ligne 37 - ligne 55 * * page 3, ligne 8 - ligne 11 * * page 3, ligne 17 - ligne 18; exemples 1-16 * ---- -/-- | 1,9-11 | |

DOMAINES TECHNIQUES RECHERCHES (Int.Cl.5)

C12P
C12N
C09K

Le présent rapport a été établi pour toutes les revendications

| Lieu de la recherche | Date d'achèvement de la recherche | Examinateur |
|---|---|---|
| LA HAYE | 16 Mai 1994 | MONTERO LOPEZ B. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C01)

13

Office européen
des brevets

## RAPPORT DE RECHERCHE EUROPEENNE

Numero de la demande

**EP 94 40 0247**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| O,A | ACS SYMPOSIUM SERIES, vol.532, 5 Avril 1992 pages 253 - 265 CHRISTINE NOIK ET AL. 'Characterization of xanthan-protein complexes obtained with various fermentation conditions' * page 253, alinéa 1 -alinéa 3 * * page 255, alinéa 2 * * page 256, alinéa 4; tableaux II,III * * page 260, alinéa 5 - page 261, alinéa 1; figure 4 * * page 261, alinéa 6 -dernier alinéa * | 1-11 | |
| | | | **DOMAINES TECHNIQUES RECHERCHES (Int.Cl.5)** |

Le présent rapport a été établi pour toutes les revendications

| Lieu de la recherche | Date d'achèvement de la recherche | Examinateur |
|---|---|---|
| LA HAYE | 16 Mai 1994 | MONTERO LOPEZ B. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)